# EUROPEAN PATENT APPLICATION

(11) **EP 1 026 258 A2**
(43) Date of publication of application: **09.08.2000**
(21) Application number: 00102106.2
(22) Date of filing: 03.02.2000
(51) Int. Cl.: C12Q 1/68

(54) **Multiplex genotyping of populations of individuals**

(30) Priority: 05.02.1999 US 245774
(71) Applicant: Affymetrix, Inc. (a California Corporation), Santa Clara, CA 95051 (US)
(72) Inventor:
(74) Representative: UEXKÜLL & STOLBERG

(57) **Abstract**

This invention provides methods for generating polymorphic profiles for many polymorphic markers in many individuals in a population. The methods involve performing multiplex amplification of the markers in many nucleic acid samples from each of many individuals to produce multiple amplification products. The resulting multiplex amplification products are applied to a substrate to create an array. Then, in one embodiment in a series of iterative passes, pairs of probes that detect both alleles of a marker are hybridized to each amplification product in the array to identify the alleles the individuals have for the marker.

## Description

### CROSS-REFERENCE TO RELATED APPLICATION

Not applicable.

### FEDERALLY SPONSORED RESEARCH OR DEVELOPMENT

Not applicable.

### FIELD OF THE INVENTION

This invention is directed to the fields of genetics, biochemistry and medical diagnostics and, in particular, to materials and methods for rapidly determining genotypes of many individuals.

### BACKGROUND OF THE INVENTION

Genotyping involves determining the identity of alleles for a gene or polymorphic marker possessed by an individual. Genotyping of individuals and populations has many uses. Genetic information about an individual can be used for diagnosing the existence or predisposition to conditions to which genetic factors contribute. Many conditions result not from the influence of a single allele, but involve the contributions of many genes. Therefore, determining the genotype for several genes can be useful for diagnosing complex genetic conditions. Genotyping of many loci from a single individual also can be used in forensic applications, for example, to identify an individual based on biological samples from the individual.

Genotyping of populations is useful in population genetics. For example, the tracking of frequencies of various alleles in a population can provide important information about the history of a population or its genetic transformation over time.

Thousands of polymorphisms in the human genome already have been identified. The identification of polymorphisms will accelerate as the human genome is completely sequenced. This makes possible the generation of polymorphic profiles containing genotypes for many genes in an individual. At present, however, no tools exist to rapidly generate polymorphic profiles for thousands of markers in thousands of individuals.

### SUMMARY OF THE INVENTION

This invention provides materials and methods for rapidly and simultaneously performing genotypic analysis for many genetic markers on many individuals. In performing the method, a nucleic acid sample is obtained from the individuals to be genotyped. Each sample is subjected to multiplex amplification to amplify segments containing the genetic markers to be examined. If necessary, each sample can be divided into fractions and a different multiplex amplification can be performed on each fraction. The products of the multiplex amplification are applied to a solid substrate in discrete locations (features) for interrogation. Each feature is interrogated to detect an allele of the amplified genetic markers. In one embodiment, the features are interrogated by contacting the features with labeled, allele-specific nucleic acid probes, and determining whether the probe hybridized to the amplification product at the feature. By repeating the process for many genetic markers, a genetic profile for many markers in the population of the individuals is developed. Using technologies to immobilize many amplification products rapidly and in a small area, this protocol can genotype at least 50,000 polymorphic markers for at least 25,000 individuals.

In one aspect this invention provides a method of detecting a polymorphic form of one and, preferably, more than one, polymorphic marker in a plurality of individuals. The method comprises a) producing a plurality of amplification products by performing a multiplex amplification on a nucleic acid sample from each of a plurality of individuals, each multiplex amplification amplifying a plurality of nucleic acid segments, each segment comprising a polymorphic marker characterized by at least two polymorphic forms; b) applying each amplification product to a discrete region of a substrate; and c) detecting the presence or absence of at least one polymorphic form of at least one polymorphic marker in each amplification product

In another embodiment the step of detecting comprises detecting in a plurality of sequential detection steps the presence or absence of a polymorphic form of a plurality of different polymorphic markers in each amplification product on the substrate. In one embodiment of the method step a) comprises dividing each sample into a plurality of fractions and performing a multiplex amplification on different polymorphic markers in each of the fractions. In another embodiment the step of detecting comprises detecting a nucleic acid probe hybridized to a segment comprising the polymorphic marker. In another embodiment the method further comprises the step of: d) generating, for the plurality of individuals, a value set indicating the presence or absence of the polymorphic form, whereby the value set determines a polymorphic profile for the individuals.

In another aspect this invention provides a kit comprising a) a plurality of primer pairs, each pair having sequences for amplifying a nucleic acid segment, wherein each segment comprises a different polymorphic marker characterized by at least two polymorphic forms; and a set of allele-specific nucleic acid probes, wherein the set comprises, for each polymorphic marker, at least one probe that specifically hybridizes to a polymorphic form of the polymorphic marker. In one embodiment the kit further comprises a substrate having a surface suitable for immobilizing the nucleic acid segments in an array. In another embodiment of the kit the set of probes comprises, for each polymorphic marker, a pair of allele-specific probes, wherein each probe of the pair specifically hybridizes to an alternative, exclusively distinguishable polymorphic form of the polymorphic marker. In another embodiment the each probe of the pair comprises a fluorescent label that emits light of a different wavelength.

In another aspect this invention provides a kit comprising: a) an array of amplification products, wherein each amplification product is the product of a multiplex amplification on a nucleic acid sample from each of a plurality of individuals, wherein each amplification product comprises a plurality of amplified nucleic acid segments, wherein each segment comprises a polymorphic marker characterized by at least two polymorphic forms; and b) a set of allele-specific nucleic acid probes, wherein the set comprises, for each amplification product, at least one probe that specifically hybridizes to a polymorphic form of at least one polymorphic marker of an amplified segment

### BRIEF DESCRIPTION OF THE DRAWINGS

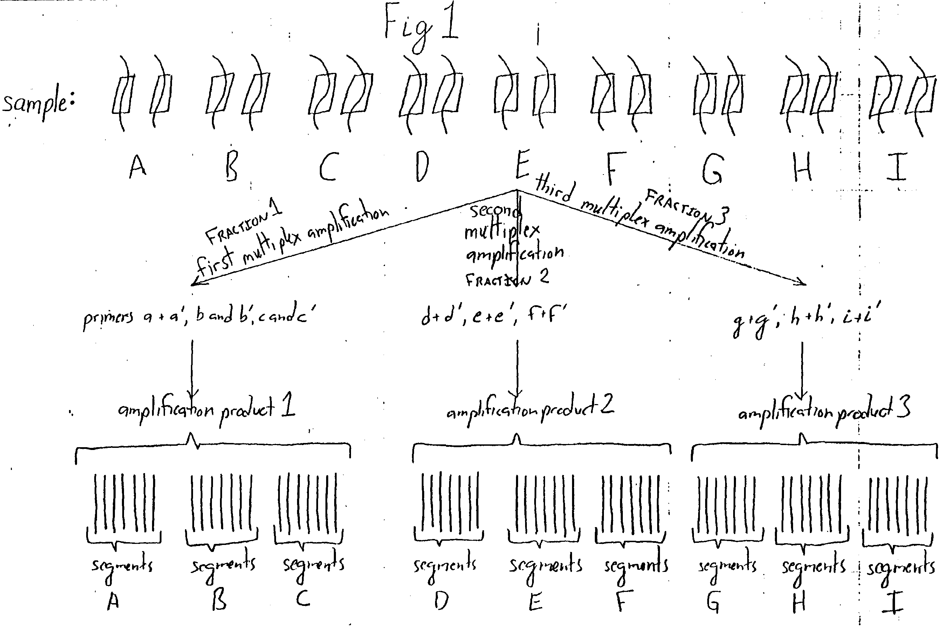

Fig. 1 shows a first step in the method of this invention: A nucleic acid sample from an individual is divided into three fractions, each of which is subjected to a multiplex amplification of polymorphic markers.

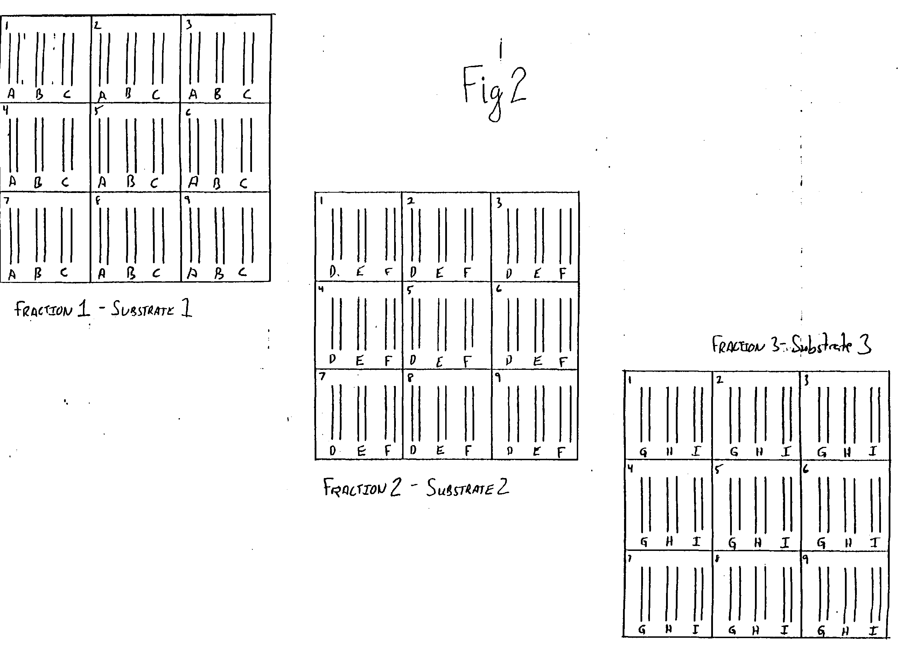

Fig. 2 shows a second step in the method of this invention: Applying the amplification products to an array. In this figure, the set of products from the first multiplex amplification on the individuals is applied to a first substrate, the set of products from the second multiplex amplification on the individuals is applied to a second substrate, and the set of products from the third multiplex amplification on the individuals is applied to a third substrate.

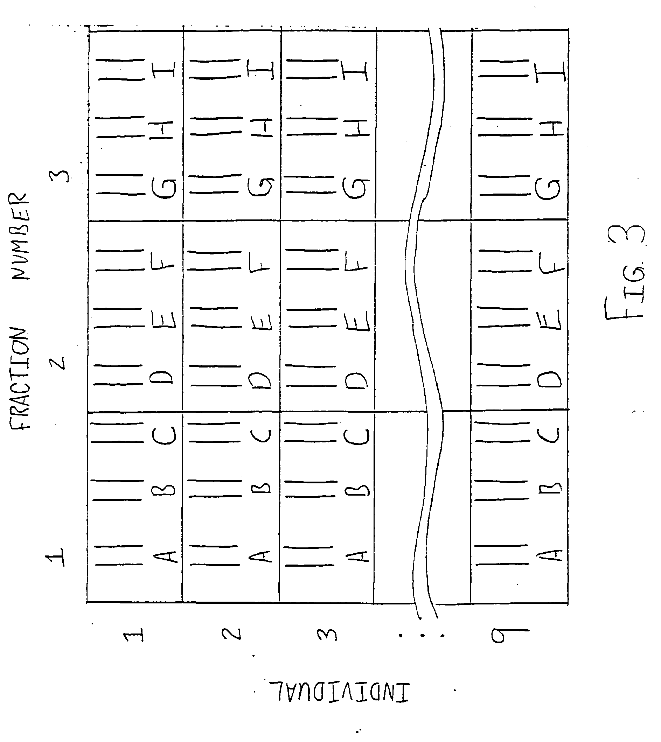

Fig. 3 shows an alternative version of the second step in the method of this invention in which the products of all multiplex amplifications from an individual are applied to different features on a substrate.

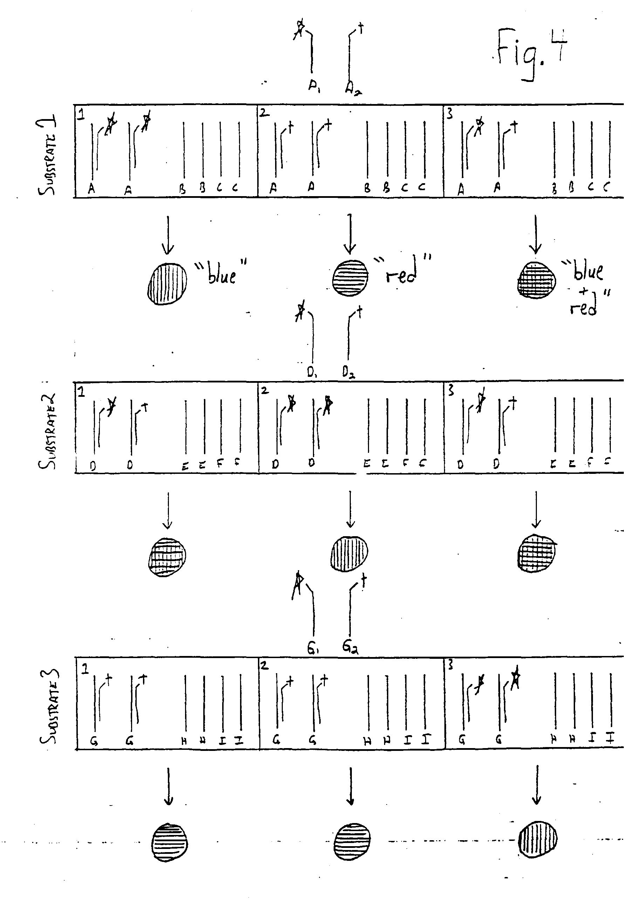

Fig. 4 shows a third step of this invention, detecting the presence or absence of a polymorphic form of a marker for each individual. In this case, one marker on each of three substrates is probed.

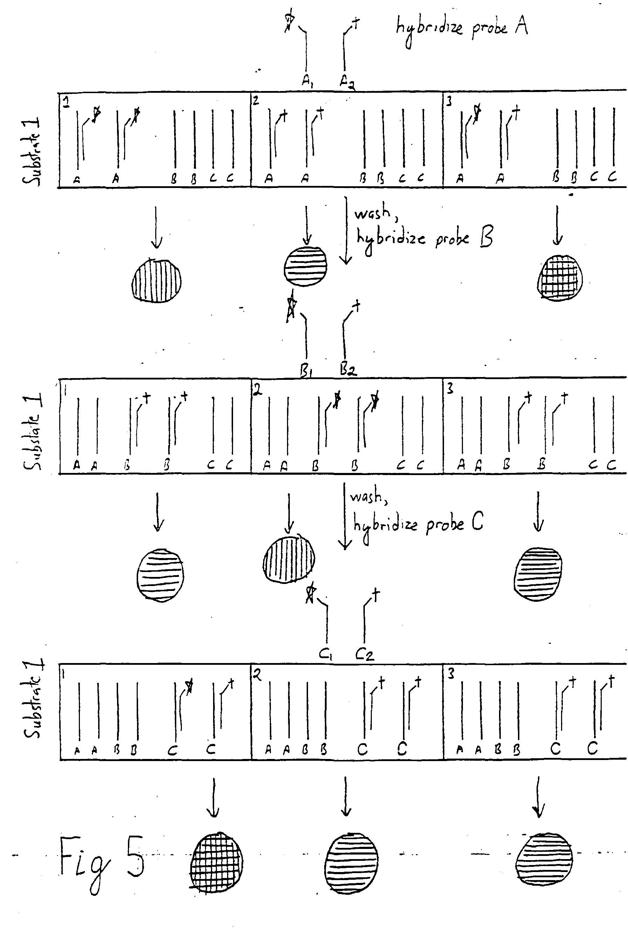

Fig. 5 shows a three-fold iteration of the detection procedure on a single substrate.

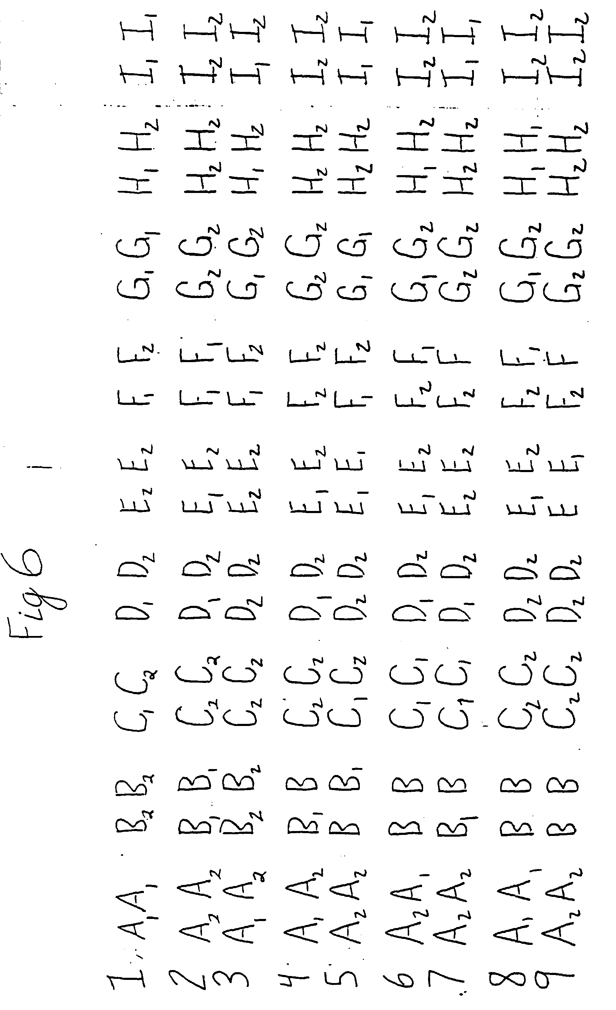

Fig. 6 shows the hypothetical generation of a multiplex polymorphic profile for markers A-I in individuals 1-9 in a population. Each multiplex polymorphic profile for an individual indicates which of two polymorphic forms (alleles), designated 1 or 2, the individual possesses for each of the markers. Because the individuals in this example are diploid, the presence or absence of each form of a marker provides a genotype for the marker.
Fig. 7A illustrates an example of a computer system used to execute software that can be used to analyze data generated by the present invention. The Figure shows a computer system 1 which includes a monitor 3, screen 5, cabinet 7, keyboard 9, and mouse 11. Mouse 11 may have one or more buttons such as mouse buttons 13. Cabinet 7 houses a CD-ROM drive 15 and a hard drive (not shown) that may be utilized to store and retrieve computer programs including code incorporating the present invention. Although a CD-ROM 17 is shown as the computer readable storage medium, other computer readable storage media including floppy disks, DRAM, hard drives, flash memory, tape, and the like may be utilized. Cabinet 7 also houses familiar computer components (not shown) such as a processor, memory, and the like.

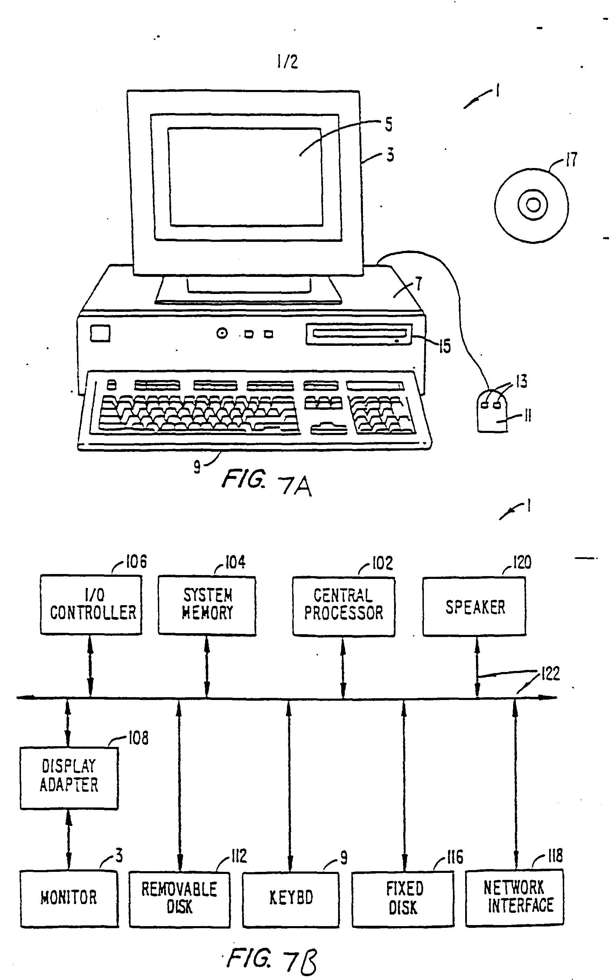

Fig. 7B shows a system block diagram of computer system 1 used to execute software that can be used to analyze data generated by the present invention. As in the previous figure, computer system 1 includes monitor 3 and keyboard 9. Computer system 1 further includes subsystems such as a central processor 102, system memory 104, I/O controller 106, display adapter 108, removable disk 112, fixed disk 116, network interface 118, and speaker 120. Removable disk 112 is representative of removable computer readable media like floppies, tape, CD-ROM, removable hard drive, flash memory, and the like. Fixed disk 116 is representative of an internal hard drive, DRAM, or the like. Other computer systems suitable for use with the present invention may include additional or fewer subsystems. For example, another computer system could include more than one processor 102 (i.e., a multi-processor system) or memory cache.

### DETAILED DESCRIPTION OF THE INVENTION

### I. DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., DICTIONARY OF MICROBIOLOGY AND MOLECULAR BIOLOGY (2d ed. 1994); THE CAMBRIDGE DICTIONARY OF SCIENCE AND TECHNOLOGY (Walker ed., 1988); THE GLOSSARY OF GENETICS, 5TH ED., R. Rieger et al. (eds.), Springer Verlag (1991); and Hale & Marham, THE HARPER COLLINS DICTIONARY OF BIOLOGY (1991). As used herein, the following terms have the meanings ascribed to them unless specified otherwise.

"Polymorphism" refers to the occurrence of two or more alternative nucleotide sequences at a particular genetic locus in the genome of a population.

"Polymorphic form" or "allele" refers to alternative forms of a polymorphism that are exclusively distinguishable in an assay.

"Polymorphic marker" or "site" refers to a genetic locus at which a polymorphism occurs. Preferred markers have at least two polymorphic forms, each occurring at frequency of greater than 1%, and more preferably greater than 10% or 20% of a selected population. A genetic locus may be as small as one base pair, if the polymorphism is a nucleotide substitution or deletion, or many base pairs if the polymorphism is, e.g., deletion, inversion or duplication of part of a chromosome. Polymorphic markers include, e.g., restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. One identified allelic form is arbitrarily designated as a the reference form and other allelic forms are designated as alternative or variant alleles. The allelic form occurring most frequently in a selected population is sometimes referred to as the wild-type form. Diploid organisms may be homozygous or heterozygous for allelic forms. A di-allelic polymorphism has two forms. A tri-allelic polymorphism has three forms.

A single nucleotide polymorphism (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

"Polymorphic profile" refers to a value set indicating, for at least one polymorphic marker in an individual, the presence or absence of a form of the polymorphic markers. For example, a polymorphic profile can provide a genotype of an individual for a plurality of genes (e.g., A₁A₁, B₂B₂, C₁C₂,...). A polymorphic profile also can provide information about one polymorphic form of a plurality of markers (e.g., A₁+, B₁-, C₂+).

"Multiplex amplification" refers to the amplification of at least two different nucleic acid segments in a single amplification reaction. In preferred embodiments, multiplex amplification involves the amplification of at least 10 different nucleic acid segments, at least 100 different nucleic acid segments or at least 250 different nucleic acid segments.

"Amplification product" refers to a collection of amplified nucleic acid segments produced in an amplification reaction.

"Amplification" refers to any means by which a nucleotide sequence of a parent molecule is copied and thus expanded into a larger number of nucleic acid molecules, e.g., by reverse transcription, polymerase chain reaction, and ligase chain reaction.

"Nucleic acid" refers to a polymer composed of nucleotide units. Nucleic acids include naturally occurring nucleic acids, such as deoxyribonucleic acid ("DNA") and ribonucleic acid ("RNA"), and nucleic acid analogs. Nucleic acid analogs include polymers of nucleotides that include non-naturally occurring bases. Nucleic acid analogs also include nucleotide polymers in which nucleotides are attached through linkages other than phosphodiester bonds. Thus, nucleotide analogs include, for example and without limitation, phosphorothioates, phosphorodithioates, phosphorotriesters, phosphoramidates, boranophosphates, methylphosphonates, chiral-methyl phosphonates, 2-O-methyl ribonucleotides, peptide-nucleic acids (PNAs), and the like. Such nucleic acids can be synthesized, for example, using an automated DNA synthesizer. "Oligonucleotide" typically refers to short nucleic acids, generally having no more than about 100 nucleotides. It will be understood that when a nucleotide sequence is represented by a DNA sequence (i.e., A, T, G, C), this also includes an RNA sequence (i.e., A, U, G, C) in which "U" replaces "T." "Nucleic acid segment" refers to a segment of a larger nucleic acid created by, e.g., fragmentation or amplification.

"Substrate" refers to a solid support capable of being divided into a plurality of features on which an amplification product can be immobilized. Substrates include, without limitation, paper, glass, nitrocellulose, silicon wafers and polymeric materials such as plastics, or gels.

"Feature" refers to an addressable location of a substrate to which targets have been applied.

"Primer" refers to a nucleic acid that is capable of specifically hybridizing to a designated nucleic acid template and providing a point of initiation for synthesis of a complementary nucleic acid. Such synthesis occurs when the nucleic acid primer is placed under conditions in which synthesis is induced, i.e., in the presence of nucleotides, a complementary nucleic acid template, and an agent for polymerization such as DNA polymerase. A primer is typically single-stranded, but may be double-stranded. Primers are typically deoxyribonucleic acids, but a wide variety of synthetic and naturally occurring primers are useful for many applications. A primer is complementary to the template to which it is designed to hybridize to serve as a site for the initiation of synthesis, but need not reflect the exact sequence of the template. In such a case, specific hybridization of the primer to the template depends on the stringency of the hybridization conditions. Primers can be labeled with, e.g., chromogenic, radioactive, or fluorescent moieties and used as detectable moieties. Primers generally will be at least 7 nucleotides long and, more preferably, about 10-25 nucleotides long.

"Probe," when used in reference to a nucleic acid, refers to a nucleic acid that is capable of specifically hybridizing to a designated sequence of another nucleic acid. A probe specifically hybridizes to a target complementary nucleic acid, but need not reflect the exact complementary sequence of the template. In such a case, specific hybridization of the probe to the target depends on the stringency of the hybridization conditions. Probes can be labeled with, e.g., chromogenic, radioactive, or fluorescent moieties and used as detectable moieties. A probe generally will be at least 8 nucleotides long and, more generally 10-25 nucleotides.

A first nucleic acid "specifically hybridizes" to a second nucleic acid if, under selected hybridization conditions, the first nucleic acid hybridizes to the second nucleic acid in a mixture of nucleic acids so as to allow detection of the second nucleic sequence and discrimination between the second nucleic acid and other nucleic acids in the mixture. Thus, for example, a perfectly complementary probe will specifically hybridize to a target sequence even under hybridization conditions that are not highly stringent, while a mismatch probe, i.e., a probe whose sequence is not perfectly complementary with the target sequence, generally will require more stringent conditions to hybridize with the target sequence in a discriminatory fashion.

The stringency of selected hybridization conditions depends on many factors including, e.g., temperature, ionic strength, pH. An extensive guide to the hybridization of nucleic acids is found in Tijssen, TECHNIQUES IN BIOCHEMISTRY AND MOLECULAR BIOLOGY--HYBRIDIZATION WITH NUCLEIC PROBES, "Overview of principles of hybridization and the strategy of nucleic acid assays" (1993). Generally, "stringent conditions" are selected to be about 5°-10° C lower than the thermal melting point (Tm) for the specific sequence at a defined ionic strength pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30° C for short probes (e.g., 10 to 50 nucleotides) and at least about 60° C for long probes (e.g., greater than 50 nucleotides). Stringent conditions may also be achieved with the addition of de-stabilizing agents such as formamide. For selective or specific hybridization, a positive signal is at least two times background, preferably 10 times background hybridization. For example, conditions of 5X SSPE (750 mM NaCl, 50 mM NaPO₄, 5 mM EDTA, pH 7.4) and a temperature of 25-30° C are suitable for allele-specific probe hybridizations.

"Moderately stringent hybridization conditions" include hybridization in a buffer of 40% formamide, 1 M NaCl, 1% SDS at 37° C, and a wash in 1X SSC at 45° C. A positive hybridization is at least twice background. Those of ordinary skill will readily recognize that alternative hybridization and wash conditions can be utilized to provide conditions of similar stringency.

"Allele-specific probe" refers to a nucleic acid probe that specifically hybridizes to a nucleic acid segment comprising an allelic form of a polymorphic marker. For example, if a polymorphic marker is characterized by polymorphic forms A₁ and A₂, an allele-specific probe is a probe that specifically hybridizes either to a nucleic acid segment comprising A₁ or to a nucleic acid segment comprising A₂.

"Detecting" refers to determining the presence, absence, or amount of an analyte in a sample, and can include quantifying the amount of the analyte in a sample.

"Label" or "detectable moiety" refers to a composition detectable by spectroscopic, photochemical, biochemical, immunochemical, or chemical means. Useful labels include, for example, ³²P, ³⁵S, fluorescent dyes, electron-dense reagents, enzymes (e.g., as commonly used in an ELISA), biotin-streptavadin, dioxigenin, haptens and proteins for which antisera or monoclonal antibodies are available, or nucleic acid molecules with a sequence complementary to a target. A label often generates a measurable signal, such as a radioactive, chromogenic, or fluorescent signal, that can be used to quantify the amount of bound detectable moiety in a sample. A label can be incorporated in or attached to a primer or probe either covalently, or through ionic, van der Waals or hydrogen bonds, e.g., incorporation of radioactive nucleotides, or biotinylated nucleotides that are recognized by streptavadin. A label may be directly or indirectly detectable. Indirect detection can involve the binding of a second directly or indirectly detectable moiety to the label. For example, the label can be the ligand of a binding partner, such as biotin, which is a binding partner for streptavadin, or a nucleotide sequence, which is the binding partner for a complementary sequence, to which it can specifically hybridize. The binding partner may itself be directly detectable, for example, an antibody may be itself labeled with a fluorescent molecule. The binding partner also may be indirectly detectable, for example, a nucleic acid having a complementary nucleotide sequence can be a part of a branched DNA molecule that is in turn detectable through hybridization with other labeled nucleic acid molecules. (See, e.g., PD. Fahrlander and A. Klausner, *Bio/Technology* (1988) 6:1165.) Quantitation of the signal is achieved by, e.g., scintillation counting, densitometry, or flow cytometry.

"Plurality" means at least two.

### II. METHODS OF RAPIDLY DETERMINING MULTIPLE POLYMORPHIC PROFILES

### A. Multiplex Amplification Of Nucleic Acid Samples From Population Members

### 1. Introduction

A first step in the method of detecting polymorphic forms in a plurality of individuals is performing multiplex amplification on a nucleic acid sample from each individual to be genotyped. If the number of genetic markers is conveniently within the capacity of a single multiplex amplification reaction, the entire amplification can be carried out on the single sample from each individual. However, if many hundreds or thousands of markers are to be examined, the nucleic acid samples typically are divided into fractions, and each fraction is subjected to a multiplex amplification. The amplifications, together, amplify segments containing all the genetic markers to be examined. For example, performing 100-plex amplifications on each of 100 fractions would amplify 10,000 markers. The result of the multiplex amplification is the creation of a set of amplification products containing amplified nucleic acid segments for each of the genetic markers. The amplification product of a particular segment could contain one form of the polymorphic marker in a haploid individual, two different forms in a diploid individual or three different forms for a triploid individual, depending upon the genotype of the individual (e.g., homozygous or heterozygous). When a sample from an individual has been divided into fractions for multiplex amplification, the amplification products from the fractions can be pooled to form a single sample (or a few samples) before testing, if desired.

### 2. Individuals

The individuals generally will be individuals from a population of organisms. This includes populations of viruses, single-celled organisms (e.g., prokaryotes or eukaryotes), animals or plants. Animal populations include vertebrates, mammals, primates and humans. Plants include agriculturally important plants such as grains (e.g., wheat rice and maize), vegetables and fruits.

The population also can be a population of cells from a cell culture. This includes, for example, metastatic cells or cells tat have been subject to mutagenesis.

The number of individuals in the population to be profiled is a plurality, generally at least 100, at least 1000, at least 10,000 or at least 25,000.

### 3. Nucleic acid samples

Polymorphisms are detected in a sample comprising nucleic acid from an individual being analyzed. For assays of genomic DNA, virtually any biological sample is suitable. For example, convenient tissue samples from mammals include whole blood, semen, saliva, tears, urine, fecal material, sweat, buccal, skin, and hair. For assays of cDNA or mRNA, the tissue sample must be obtained from an organ in which the target nucleic acid is expressed. For example, if the target nucleic acid is a cytochrome P450, the liver is a suitable source.

### 4. Multiplex amplification

The methods of this invention involve amplification of nucleic acids from target samples. Several methods are known in the art for amplifying nucleic acid segments.

A preferred method is the polymerase chain reaction, PCR. See generally PCR TECHNOLOGY: PRINCIPLES AND APPLICATIONS FOR DNA AMPLIFICATION (ed. H.A. Erlich, Freeman Press, NY, NY, 1992); PCR PROTOCOLS: A GUIDE TO METHODS AND APPLICATIONS (eds. Innis, et al., Academic Press, San Diego, CA, 1990); Mattila et al., *Nucleic Acids Res.* 19, 4967 (1991); Eckert et al., *PCR Methods and Applications* 1, 17 (1991); PCR (eds. McPherson et al., IRL Press, Oxford); and U.S. Patent 4,683,202 (Mullis). Primers for amplification are selected to flank a region of interest in a target sample. For example, primers can be designed to be flank a known site of variation and a few bases on either side, or to flank an exon, or to flank a whole coding sequence or gene.

Other suitable amplification methods include the ligase chain reaction (LCR) (see Wu and Wallace, *Genomics* 4, 560 (1989), Landegren et al., *Science* 241, 1077 (1988), transcription amplification (Kwoh et al., *Proc. Natl. Acad. Sci. USA,* 86, 1173 (1989)), and self-sustained sequence replication (Guatelli et al., *Proc. Nat. Acad. Sci. USA,* 87, 1874 (1990)) and nucleic acid based sequence amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively.

One version of multiplex amplification is described in Wang et al., *Science,* 280:1077 (1998), footnote 26. According to this method, multiplex PCR is performed by using multiple PCR primer pairs in a single reaction. Specifically, multiplex PCR reactions are performed in a 50 µl volume containing 100 ng of the subject's DNA, 0.1 to 0.2 µM of each primer, 1 unit of AmpliTaq Gold (Perkin-Elmer), 1 mM deoxynucleotide triphosphates (dNTPs), 10 mM tris-HCl (pH 8.3), 50 mM KCl, 5 mM MgCl₂ and 0.001% gelatin. Thermocycling is performed on a Tetrad (MJ Research), with initial denaturation at 96°C for 10 min followed by 30 cycles of denaturation at 96° C for 30 seconds, primer annealing at 55° C for 2 min, and primer extension at 65° C for 2 min. After 30 cycles, a final extension reaction was carried out at 65°C for 5 min. If the resulting PCR products are small, it will be unnecessary to fragment them. The PCR products are then labeled with biotin in a standard PCR reaction, by using T7 and T3 primers with biotin labels at their 5'-ends. The reaction is performed with 1 µl of template DNA, 0.1 to 0.2 µM labeled primer, 1 unit of AmpliTaq Gold (Perkin-Elmer), 100 µM dNTPs, 10 mM tris-HCl (pH 8.3), 50 mM KCl, 1.5 mM MgCl₂, and 0.001% gelatin. Thermocycling is performed with initial denaturation at 96°C for 10 min followed by 25 cycles of denaturation at 96° C for 30 seconds, primer annealing at 52°C for 1 min, and primer extension at 72°C for 1 min. After 25 cycles, a final extension reaction is carried out at 72° C for 5 min. The PCR products from the various multiplex reactions for an individual can be together.

### B. Preparing Substrate-bound Arrays Of Amplification Products

### 1. Introduction

A second step in the method involves immobilizing the amplification products on a solid substrate in discrete, addressable locations. These locations are referred to as "features." Thus, immobilization of the amplification products produces an array of features. If the polymorphic markers have been amplified in a single multiplex amplification reaction for each individual, or if the products of several multiplex amplification reactions for an individual are pooled into a single sample, then the samples from all the individuals can be arranged on a single substrate, or as many substrates as are necessary to accommodate all the individuals. The array can take any desired shape. However, orthogonal arrays of rows and columns frequently are easy to manipulate and keep track of.

If the amplified markers for an individual are divided among several fractions, then there are several ways to arrange the samples. Preferably, the multiplex amplification reactions are set up so as to amplify the same polymorphic markers from a fraction from each of the individuals. In a preferred method, the set of products containing the same amplified genetic markers for all the individuals are immobilized on one or more substrates, so that each substrate contains only fractions containing the same amplified markers. This arrangement can simplify application of detection probes to the substrate. The number of individuals spotted on an array depends upon the capacity of the substrate and the feature technology. Thus, if samples can be spotted one millimeter apart, then amplification products of 10,000 individuals can be placed in an array of 10 cm x 10 cm. Also, the number of amplified markers immobilized at any feature is a function of the power of the multiplex reaction and/or the ability to pool different amplification products into a single sample.

### 2. Nucleic acid arrays

Several strategies are available for immobilizing amplification products on solid supports. The nucleic acids can be directly attached to a substrate that binds nucleic acids, such as paper, glass or nitrocellulose. Alternatively, they can be attached through linkers, for example, oligonucleotide linkers, attached to a substrate.

### a. Spotting methods

In one version, a substrate is provided which has an array of discrete reaction regions, usually separated from one another by inert regions. In one embodiment, a first nucleic acid solution is spotted on a first region of a suitably derivatized substrate. Thereafter, a second nucleic acid sample is spotted on a second region, a third nucleic acid sample is spotted on a third region and so on, until a number of the regions each have an amplification product located therein.

In another strategy, the amplification products are prepared in an array of sample wells, e.g., a 96-well plate. An array of pins is dipped into the wells, picking up liquid containing the oligonucleotides. Then the pins are pressed against a substrate that binds the nucleic acids such as, for example, paper, glass or nitrocellulose. The nucleic acids in the sample are thus immobilized in an array. Thus, for example, each well of the 96-well plate could contain the product of the same multiplex amplification reaction for each of 96 different individuals in the population. The amplification products from each well are then spotted at a different location on a substrate.

### b. Spraying methods

Another strategy involves the use of an array of capillary tubes that contain the liquid samples. When the capillary tubes are touched on the surface of a substrate, a drop of the sample is deposited there. One version of this method is described in WO 95/35505 (Shalon et al.).

Another strategy takes advantage of ink-jet technology. Such ink-jets are commonly used in printers in which tiny ink drops are sprayed onto specific locations on a substrate, such as paper. According to this strategy, capillary tubes are connected from the nozzle end into wells that contain the nucleic acid samples. This technology can create very dense arrays of immobilized nucleic acid samples.

### c. Hybridizing to oligonucleotide anchors

In another strategy, the amplification process involves supplying the amplification product with a nucleic acid sequence tag. This is accomplished, for example, by using primers that include the tag along with the complementary portion that hybridizes to the target. The tags could be specific for an individual, or specific for the particular multiplex amplification. For example, individual 1 may be amplified to contain a first sequence tag. Individual 2 may be amplified to contain a second sequence tag. Individual 3 may be amplified to contain a third sequence tag.

Then, an array of sequence-specific oligonucleotides having the complement of the tag are assembled on a substrate at discrete, addressable locations. When the amplified segments from a fraction are added to the array, the sequence tags hybridize with the complementary anchors at specific locations. In this way, the fragments will sort themselves on the array to specific locations.

The anchors can be localized by a version of spatially directed oligonucleotide synthesis.

### i. Spatially directed oligonucleotide synthesis-Version 1

In one embodiment substrate-bound nucleic acids are immobilized at specific locations by light-directed oligonucleotide synthesis. The pioneering techniques of this method are disclosed in U.S. Patent No. 5,143,854 (Pirrung et al.), U.S. Patent 5,571,639 (Hubbell et al.), U.S. Patent 5,744,101 (Fodor et al.) and U.S. Patent 5,489,678 (Fodor et al.). In a basic strategy of this process, the surface of a solid support modified with linkers and photolabile protecting groups is illuminated through a photolithographic mask, yielding reactive hydroxyl groups in the illuminated regions. A 3^{'}-O-phosphoramidite-activated deoxynucleoside (protected at the 5'-hydroxyl with a photolabile group) is then presented to the surface and coupling occurs at sites that were exposed to light. Following the optional capping of unreacted active sites and oxidation, the substrate is rinsed and the surface is illuminated through a second mask, to expose additional hydroxyl groups for coupling to the linker. A second 5'-protected, 3'-O-phosphoramidite-activated deoxynucleoside (C-X) is presented to the surface. The selective photodeprotection and coupling cycles are repeated until the desired set of products is obtained. Photolabile groups are then optionally removed and the sequence is, thereafter, optionally capped. Side chain protective groups, if present, are also removed. Since photolithography is used, the process can be miniaturized to generate high-density arrays of oligonucleotide probes.

In the present invention, linkers can be built over the surface of substrate and the samples can be coupled at various locations by activating the groups at that location using the lithographic techniques just described.

This general process can be modified. For example, the nucleotides can be natural nucleotides, chemically modified nucleotides or nucleotide analogs, as long as they have activated hydroxyl groups compatible with the linking chemistry. The protective groups can, themselves, be photolabile. Alternatively, the protective groups can be labile under certain chemical conditions, e.g., acid. In this example, the surface of the solid support can contain a composition that generates acids upon exposure to light. Thus, exposure of a region of the substrate to light generates acids in that region that remove the protective groups in the exposed region. Also, the synthesis method can use 3'-protected 5'-O-phosphoramidite-activated deoxynucleoside. In this case, the oligonucleotide is synthesized in the 5' to 3' direction, which results in a free 5' end.

The general process of removing protective groups by exposure to light, coupling nucleotides (optionally competent for further coupling) to the exposed active sites, and optionally capping unreacted sites is referred to herein as "light-directed nucleotide coupling."

### ii. Spatially directed oligonucleotide synthesis-Version 2

Another strategy is described in United States patent 5,667,195 (Winkler et al.). According to this method, a series of channels, grooves, or spots are formed on or adjacent a substrate. Reagents are selectively flowed through or deposited in the channels, grooves, or spots, forming an array having different compounds -- and in some embodiments, classes of compounds -- at selected locations on the substrate. There are two main versions of this method.

In one version, a block having a series of channels, such as grooves, on a surface thereof is utilized. The block is placed in contact with a derivatized glass or other substrate. In a first step, a pipettor or other delivery system is used to flow selected reagents to one or more of a series of apertures connected to the channels, or place reagents in the channels directly, filling the channels and "striping" the substrate with a first reagent, coupling the nucleic acids thereto. The block is then translated or rotated, again placed on the substrate, and the process is repeated with a second reagent, coupling a second group of monomers to different regions of the substrate. The process is repeated until a diverse set of polymers of desired sequence and length is formed on the substrate. By virtue of the process, a number of polymers having diverse monomer sequences such as peptides or oligonucleotides are formed on the substrate at known locations.

In another version, a series of micro-channels or micro grooves are formed on a substrate, along with an appropriate array of microvalves. The channels and valves are used to flow selected reagents over a derivatized surface. The microvalves are used to determine which of the channels are opened for any particular coupling step.

Similarly, various locations can be activated to couple with the nucleic acid segments in the amplification products so as to immobilize those products.

### C. Detecting The Presence Of Polymorphic Forms Of Markers For Each Individual In The Population

### 1. Introduction

A third step in the process for determining polymorphic forms of a marker for a population of individuals involves detecting the presence or absence of a polymorphic form of at least one marker for each individual in the population. This step is simplified in present invention by the provision of a substrate that contains immobilized amplification product for all the members of the population. The substrate allows one to probe for any of the amplified markers in all the individuals concurrently within the confined space of the substrate.

In a general version, the method involves detecting the presence or absence of a single polymorphic form of one or more markers for a plurality of individuals in the population. For example, a multiplex amplification reaction may amplify markers A, B and C in a nucleic acid sample of the individuals. These markers may have two polymorphic forms each, e.g., A₁ and A₂, B₁ and B₂, and C₁ and C₂. The amplification product for each individual will contain amplified segments containing the markers. The particular polymorphic forms in the amplified segments depend, of course, on each individual's genotype. The practitioner can probe the amplification product of each individual to detect the presence or absence of allele A₁. This process can then be repeated for another allele of the same or of a different marker. For example, after probing the amplification products for the presence or the absence of allele A₁, the practitioner could probe the same substrate (or another substrate on which the same amplification product has been laid down) for allele B₁.

Frequently, it will be more useful to determine the entire genotype of each individuals for the marker to be probed, i.e., the identity of all alleles possessed by the individual. For example, the practitioner could probe the amplification products of all the individuals for both alleles A₁ and A₂. In a diploid individual, the presence or absence of these alleles would indicate whether the individual is homozygous (A₁A₁ or A₂A₂) or heterozygous (A₁A₂). Again, after determining the genotype for a first marker, one could probe the array to determine the genotype for a second marker, e.g., B or C, either on the same substrate, or a different substrate blotted with the same amplification product.

### 2. Reference Sequences

Reference sequences for polymorphic markers can be obtained from computer databases such as Genbank, the Stanford Genome Center, The Institute for Genome Research and the Whitehead Institute. The latter databases are available at http://www-genome.wi.mit.edu; http://shgc.stanford.edu and http://ww.tigr.org. Reference sequences are typically from well-characterized organisms, such as human, mouse, *C. elegans, arabidopsis, Drosophila, yeast, E. coli* or *Bacillus subtilis.* A reference sequence generally is sufficiently long to specify the polymorphic marker and include the polymorphic forms. Thus, the reference sequence should be long enough to allow specific detection in any of the detection assays. For example, in hybridization assays, the reference sequence generally will be at least 8 nucleotides longs to around 50 nucleotides long. The reference sequence can be from expressed or non-expressed regions of the genome. In some methods, in which RNA samples are used, highly expressed reference sequences are sometimes preferred to avoid the need for RNA amplification. The function of a reference sequence may or may not be known. Reference sequences can also be from episomes such as mitochondrial DNA. Of course, multiple reference sequences can be analyzed independently.

A substantial number of polymorphic sites in humans and other species have been described in the published literature, and many other polymorphic sites in human genomic DNA are described in commonly owned co-pending patent applications, such as PCT/US98/04571, filed March 5, 1998. The genomic locations of these sites are known, as is the nature of the polymorphic forms occurring at the sites. Many of the known polymorphic sites occur within so-called expressed sequence tags and are therefore represented in the transcript of genomic DNA, as well as genomic DNA itself. Often, the polymorphism is found outside the coding sequence of a gene; for example, in a promoter, other regulatory sequence or an intronic sequence.

### 3. Methods Of Detecting Nucleic Acids With Specific Reference Sequences

Any method of detecting a specific nucleotide sequence immobilized to a support is contemplated here. A preferred method involves specifically hybridizing a probe to the target sequence, and detecting the hybridized probe. A hybridized probe can be detected directly, for example by mass spectrometry, or indirectly, by detecting a label associated with the probe or with hybridization.

A method of direct detection is mass spectrometry, in which a hybridized probe is desorbed from the substrate and identified based on its molecular mass (e.g., MALDI-TOF). Labeling methods, in which the presence or absence or an allele is indicated by the presence or absence of a detectable label, involve a detectable label that comes to be associated with an immobilized molecule having a specific sequence.

One label-based detection system involves detecting a specific sequence by hybridizing a probe specifically to the sequence, and detecting the hybridized probe. The hybridized can be detected directly, for example through mass spectrometry, or indirectly, through the use of a label. Three particularly useful versions of this method described below are: (1) allele-specific hybridization, (2) allele-specific extension and (3) allele-specific ligation.

In another version of label-based detection, a label is released by molecules having the sequence. For example, the immobilized molecules can be labeled. Then, one can hybridize an allele specific probe the target. If the allele is present, a double stranded molecule is created. The substrate is then subject to cleavage by a specific or non-specific endonuclease that cleaves double stranded DNA. This releases the label. Thus, a decrease in the presence of label indicates the presence of the allele.

The number of alleles that one can determine in any single assay depends upon the nature of the assay. For example, fluorescent labels exist that fluoresce in several different wavelengths that are distinguishable. If, for example, the assay system used can distinguish four different fluorescent labels, then one could detect the presence of absence of four different polymorphic forms. The practitioner can make use of this in several ways. For example, a single polymorphic marker may have multiple alleles. Assume for this example that there are four alleles, A₁, A₂, A₃ and A₄. Using four different labels, one could determine which of the four alleles is present in a single assay. Alternatively, one may use the four labels to detect two alleles each in two, different markers. For example, one could probe for alleles A₁, A₂, B₁ and B₂. Again, in a second assay on the same substrate or different substrate with the same amplification products, the practitioner could probe for two more of the amplified segments.

Finally, as discussed above, the process can involve performing many different multiplex amplification reactions on aliquots of DNA from each of the individuals. Thus, assays can be run in parallel, with one set of markers being probed on a first substrate, and a second set of markers being probed on a second substrate.

### a. Allele-specific hybridization

One method of detecting on a substrate an immobilized nucleic acid having a particular sequence is to contact the substrate with a labeled nucleic acid probe that specifically hybridizes with a nucleic acid having the sequence. The presence of the sequence is detected by detecting the presence of the label at the feature.

Accordingly, allele-specific hybridization involves hybridizing to each immobilized amplification product at least one allele-specific nucleic acid probe, wherein each allele-specific probe specifically hybridizes to a specific polymorphic form of a polymorphic marker of an amplified segment in the amplification product. In a preferred embodiment, each substrate is probed with a pair of mutually distinguishable nucleic acid probes (in the case diploid individuals), each one specific for an alternative form of a polymorphic maker. In this case, the probe pairs generally will have two distinguishable labels. For example, the labels could be fluorescent labels that fluoresce at two different wavelengths. When both labels are present, both wavelengths can be detected. By measuring the ratio of the amounts of light at each wavelength, one can determine the ratio of the amount of hybridized probe as a function of the ratio of the amount of light of each wavelength.

Probes need not be used in pairs for a single marker. For example, the practitioner may choose to use a single probe indicating the presence or absence of a chosen allele. Also, the practitioner may choose to use one probe that detects one allele of a first marker and a second probe that detects one allele of a second marker. Furthermore, because more than two probes hybridized to a feature can be distinguished, the practitioner can use more than one pair of probes, each pair directed to polymorphic forms of a different marker in an amplification product and each probe distinguishable from the others.

### b. Allele-specific ligation

Another method of determining specific nucleotide sequences is by allele-specific ligation. This method is described in some detail in U.S. patent 5,830,711 (Barany et al.). In this method, the immobilized molecules are contacted with a locus-specific probe under hybridization conditions. A locus-specific probe hybridizes to a sequence that is specific for the polymorphic marker and, therefore, possessed by all amplified fragments regardless of the particular allele. The substrate also is contacted with one or more labeled allele-specific probes. That is, these probes hybridize only fragments having the specific allele at the locus. The locus-specific and allele-specific probes are selected so that they hybridize to the target directly adjacent to one another so that their termini are contiguous. Then the substrate is contacted with a ligase. The ligase ligates nucleic acids hybridized adjacent to one another. However, it does not ligate fragments that are separated by one or more nucleotides or whose terminal nucleotides are not complementary to the target and, therefore, not hybridized to it. Thus, whenever a particular allele is present a labeled probe will be ligated to a locus specific probe hybridized at the locus. The substrates are washed under wash conditions so that an allele-specific probe will not remain hybridized to the target unless it is ligated to a locus-specific probe.

This method of detection has certain advantages over allele-specific hybridization. A longer probe provides greater sensitivity for a target molecule than a shorter probe because it will hybridize under more stringent conditions. However, under similar stringency conditions, a shorter probe is more specific for a target than a longer probe because the shorter probe will tolerate fewer mismatches in hybridizing than a longer probe. Allele-specific ligation takes advantage of both of these facts. It relies on two shorter probes to provide specificity. However, because it only ligates perfectly hybridized termini, the ligase provides the target sensitivity of longer probes.

### c. Allele-specific primer extension

Another method of determining specific nucleotide sequences is by allele-specific primer extension. In this method, each allelic form is detected through the incorporation into a primer of a specifically labeled nucleotide characteristic to the allele. For example, two (or three or four) polymorphic forms of a marker may have a different nucleotide at a particular position in the sequence. In this method, the practitioner prepares a primer that is complementary to the sequence just adjacent to the point of difference. Then one performs a primer extension reaction on the primer in the direction of the difference. However, rather than using chain extending nucleotides, the practitioner uses differently labeled chain terminating nucleotides, such as dideoxynucleotides. For example, each of the four nucleotides can be labeled with a differently colored fluorescent marker. In this case, only one nucleotide can be added to the primer on any of the amplified stands. Therefore, the identity of the nucleotide depends upon the particular polymorphic form. Thus, detection of any particular labeled nucleotide indicates the particular polymorphic form at a feature. Where the individual is heterozygous, two forms of the signal are detectable. An advantage of this method is that four different allelic variants of a single marker are detectable.

### D. Performing Hybridization Assays

In one embodiment of the invention, polymorphic forms are detected by detecting a probe hybridized to a nucleic acid segment comprising a polymorphic marker that includes the polymorphic form. Therefore, methods of performing hybridization assays is presented here.

### 1. Probes

Probes for hybridization with immobilized molecules generally will be from 8 nucleotides to about 100 nucleotides long. Preferably, probes have between about 10-50 or 15-30 nucleotides. Probes typically will be labeled with a fluorescent label, because such labels can be distinguished and can be detected in the small areas the features can attain. However, any detectable label can be used.

### 2. Carrying out hybridization assays

Hybridization assays on nucleic acid arrays can include contacting an array with a labeled sample under the selected hybridization conditions, optionally washing the array to remove un-reacted molecules, and analyzing the biological array for evidence of reaction between target molecules the probes. These steps involve handling fluids. These steps can be automated using automated fluid handling systems for concurrently performing the detection steps on the array. Fluid handling allows uniform treatment of samples in the wells. Microliter robotic and fluid-handling devices are available commercially, for example, from Tecan AG.

The array can be manipulated by a fluid-handling device. This robotic device can be programmed to set appropriate reaction conditions, such as temperature, add reagents to the array, incubate the array for an appropriate time, remove un-reacted material, wash the array substrate, add reaction substrates as appropriate and perform detection assays. The particulars of the reaction conditions are chosen depends upon the purpose of the assay, for example hybridization of a probe or attachment of a label to oligonucleotides.

If desired, the array can be appropriately packaged for use in array reader. One such apparatus is disclosed in International publication WO 95/33846 (Besemer et al.).

### 3. Detecting Signal From Probes Bound To Features

### a. Introduction

Detecting binding between a particular probe (e.g., allele-specific, allele-specific ligated or primer extended) and the amplification product in a feature on the array under specific hybridization conditions indicates that the individual to whom the feature corresponds has the polymorphic form of the marker detected by the probe. The intensity of binding between a probe and the products of the same amplification reactions can provide an indication of the genotype for the particular marker the probe is designed to distinguish for the individuals in the population. For example, a strong signal for a particular probe can indicate homozygosity, while a weak signal can indicate heterozygosity. The collection of genotypical information for each marker tested in each fraction from an individual yields a polymorphic profile for that individual. The assembly of polymorphic profiles for each individual member of the population yields a polymorphic profile of the population.

In a preferred embodiment, the process of hybridization and detection is iterated a plurality of times in order to obtain information about a plurality (preferably each) of the markers amplified in a multiplex amplification reaction. This produces information about the plurality of amplified markers for a plurality of the individuals to be genotype.

### b. Detecting fluorescently labeled probes

Determining a signal generated from a detectable label on an array requires an array reader. The nature of the array reader depends upon the particular type of label attached to the target molecules.

In one embodiment the array reader comprises a body for immobilizing the nucleic acid array. Excitation radiation, from an excitation source having a first wavelength, passes through excitation optics from below the array. The excitation optics cause the excitation radiation to excite a region of an nucleic acid array on the substrate. In response, labeled material on the sample emits radiation which has a wavelength that is different from the excitation wavelength. Collection optics, also below the array, then collect the emission from the sample and image it onto a detector. The detector generates a signal proportional to the amount of radiation sensed thereon. The signals can be assembled to represent an image associated with the plurality of regions from which the emission originated.

According to one embodiment, a multi-axis translation stage moves the nucleic acid array in order to position different areas to be scanned, and to allow different locations of an array to be interrogated. As a result, a 2-dimensional image of the nucleic acid array is obtained.

The nucleic acid array reader can include an auto-focusing feature to maintain the sample in the focal plane of the excitation light throughout the scanning process. Further, a temperature controller may be employed to maintain the sample at a specific temperature while it is being scanned. The multi-axis translation stage, temperature controller, auto-focusing feature, and electronics associated with imaging and data collection are managed by an appropriately programmed digital computer.

In one embodiment, a beam is focused onto a spot of about 2 µm in diameter on the surface of the array using, for example, the objective lens of a microscope or other optical means to control beam diameter. (See, e.g., United States patent 5,631,734 (Stern et al.)).

In another embodiment fluorescent probes are employed in combination with CCD imaging systems. Details of this method are described in United States patent 5,578,832 (Trulson et al.). In many commercially available microplate readers, typically the light source is placed above an array, and a photodiode detector is below the array. For the present methods, the light source can be replaced with a higher power lamp or laser. In one embodiment, the standard absorption geometry is used, but the photodiode detector is replaced with a CCD camera and imaging optics to allow rapid imaging of the array. A series of Raman holographic or notch filters can be used in the optical path to eliminate the excitation light while allowing the emission to pass to the detector. In a variation of this method, a fiber optic imaging bundle is utilized to bring the light to the CCD detector. In another embodiment, the laser is placed below the nucleic acid array and light directed through the transparent wafer or base that forms the bottom of the nucleic acid array. In another embodiment, the CCD array is built into the wafer of the nucleic acid array.

The choice of the CCD array will depend on the number of features in each array. If 2500 features nominally arranged in a square (50 x 50) are examined, and 6 lines in each feature are sampled to obtain a good image, then a CCD array of 300 x 300 pixels is desirable in this area. However, if an individual array has 48,400 features (220 x 220) then a CCD array with 1320 x 1320 pixels is desirable. CCD detectors are commercially available from, e.g., Princeton Instruments, which can meet either of these requirements.

The detection device also can include a line scanner, as described in United States patent 5,578,832 (Trulson et al.). Excitation optics focuses excitation light to a line at a sample, simultaneously scanning or imaging a strip of the sample. Surface-bound fluorescent labels from the array fluoresce in response to the light. Collection optics image the emission onto a linear array of light detectors. By employing confocal techniques, substantially only emission from the light's focal plane is imaged. Once a strip has been scanned, the data representing the 1-dimensional image are stored in the memory of a computer. According to one embodiment, a multi-axis translation stage moves the device at a constant velocity to continuously integrate and process data. Alternatively, galvometric scanners or rotating polyhedral mirrors may be employed to scan the excitation light across the sample. As a result, a 2-dimensional image of the sample is obtained.

In another embodiment, collection optics direct the emission to a spectrograph which images an emission spectrum onto a 2-dimensional array of light detectors. By using a spectrograph, a full spectrally resolved image of the array is obtained.

The read time for an array will depend on the photophysics of the fluorophore (i.e., fluorescence quantum yield and photodestruction yield) as well as the sensitivity of the detector. For fluorescein, sufficient signal-to-noise to read a array image with a CCD detector can be obtained in about 30 seconds using 3 mW/cm² and 488 nm excitation from an Ar ion laser or lamp. By increasing the laser power, and switching to dyes such as CY3 or CY5 which have lower photodestruction yields and whose emission more closely matches the sensitivity maximum of the CCD detector, one easily is able to read each array in less than 5 seconds.

### E. Generating Polymorphic Profiles

### 1. Introduction

Using information regarding the presence or absence of polymorphic forms of markers, one can then generate a polymorphic profile for each of the individuals in the population. The data is processed, preferably by programmable digital computer.

### 2. Data analysis

Data is most easily analyzed with the use of a programmable digital computer. (See Figs. 7A and 7B.) The computer program generally contains a readable medium that stores the codes. Certain code is devoted to memory that includes the location of each feature and the identity of the individual and the polymorphic markers at that feature. The program also can include in its memory the reference sequences of the markers. The computer also can contain code that correlates detection of a particular signal with a particular probe and the presence of hybridization with the presence of a particular allele. The computer also can contain code that receives as input hybridization data from a hybridization reaction between a probe and the segments at a particular feature. The computer also can contain code that relates the existence or extent of hybridization with the presence of a single or double copy of the allele. The computer program also can include code that receives instructions from a programmer as input.

The computer can transform the data into another format for presentation. Data analysis can include the steps of determining, e.g., fluorescent intensity as a function of substrate position from the data collected, removing "outliers" (data deviating from a predetermined statistical distribution), and calculating the relative binding affinity of the targets from the remaining data The resulting data can be displayed as an image with color in each region varying according to the light emission or binding affinity between targets and probes therein. Alternatively, the data can be presented as a list indicating each individual and the genotype for each polymorphic marker tested.

One application of this system when coupled with the CCD imaging system that speeds performance when the detection step involves hybridization of a labeled target oligonucleotide with an oligonucleotide in the array is to obtain results of the assay by examining the on- or off-rates of the hybridization. In one version of this method, the amount of binding at each address is determined at several time points after the targets are contacted with the array. The amount of total hybridization can be determined as a function of the kinetics of binding based on the amount of binding at each time point. Thus, it is not necessary to wait for equilibrium to be reached. The dependence of the hybridization rate for different oligonucleotides on temperature, sample agitation, washing conditions (e.g., pH, solvent characteristics, temperature) can easily be determined in order to maximize the conditions for rate and signal-to-noise. Alternative methods are described in United States patent 5,324,633 (Fodor et al.).

The dependence of the hybridization rate for different oligonucleotides on temperature, sample agitation, washing conditions (e.g., pH, solvent characteristics, temperature) can easily be determined in order to maximize the conditions for rate and signal-to-noise.

The results of hybridization assays performed on the array generally will be analyzed by programmable digital computer. Such a computer can store, in its memory, the identity of every amplification product, including the identity of the individual and the segments amplified in every amplification reaction. Therefore, while orthogonal arrays having individuals in rows and amplification reactions in columns (or vice versa) is preferred for ease of use, the amplification products can be put down in any arrangement, including randomly.

### EXAMPLE

The following example is offered by way of illustration, not by way of limitation. It shows a method for preparing a multiplex polymorphic profile for nine genetic markers A-I in a population of nine individuals.

### I. MULTIPLEX AMPLIFICATION

Referring to Fig. 1, each member of a population of diploid individuals has two alleles for each of nine polymorphic genetic markers: A, B, C, D, E, F, G, H and I. The particular identity of the alleles is not, at this point, identified. A DNA sample from each individual is divided into three fractions. A first fraction from each individual is subject to a first multiplex amplification reaction, in this case a three-plex amplification, using primers a and a', b and b', and c and c' to amplify segments, indicated by boxes, containing the markers. This yields a first amplification product for each individual containing amplified copies of nucleic acid segments comprising markers A, B and C.

A second fraction from each of the individuals is subject to a second multiplex amplification reaction using primers d and d', e and e' and f and f' to yield a second amplification product containing amplified copies of markers D, E and F.

A third fraction is subject to a third multiplex amplification to yield a third amplification product containing amplified copies of markers G, H and I.

### II. APPLICATION OF AMPLIFICATION PRODUCT TO SUBSTRATE

Referring to Fig. 2, the set of first amplification products for nine individuals in the population are immobilized in an orthogonal array of features on a first substrate. The second set of amplification products for all the individuals are immobilized in an orthogonal array of features on a second substrate. The third set of amplification products for all the individuals are immobilized in an orthogonal array of features on a third substrate.

The amplification products can be applied to the substrate in other arrangements, as well. For example, as shown in Fig. 3, the amplification products for each individual can be arranged in array on a single substrate. For example, the array can take the from of a grid with rows and columns. Each feature in a row can contain the product of a different multiplex amplification reaction for the same individual. Each feature in a column can contain the product of same multiplex amplification reaction on a fraction from each of the different individuals. Referring to Fig. 3, a plurality of amplification products (in this case three) from a plurality of individuals (in this case 9) are applied to discrete, addressable locations (features) on a substrate, yielding twenty-seven (3 x 9) features.

### III. PROBING MULTIPLE SUBSTRATES FOR ALLELIC FORM OF MARKER

The amplification products are now probed to determine the identity of the polymorphic form of at least one marker for each of the individuals. Employing the arrangement of amplification products shown in Fig. 2, the three substrates are probed as shown in Fig. 4. For simplicity, only individuals 1-3 are shown for each substrate. A pair of probes is chosen that is directed to one polymorphic marker in each set of amplification products. In this example, the probes are directed to polymorphic forms A₁ and A₂ of marker A in substrate 1, D₁ and D₂ of marker D in substrate 2 and G₁ and G₂ of marker G in substrate 3. At each feature, upon contact the probes hybridize to whatever polymorphic forms exist in the amplification product

For example, on substrate 1, only probe A₁ hybridizes to the amplification product for individual 1, indicating a homozygous individual for allele A₁. Only probe A₂ hybridizes to the amplification product from individual 2, indicating a homozygous individual for allele A₂. Both probes A₁ and A₂ hybridize to the amplification product from individual 3, indicating a heterozygous individual, A₁A₂.

On substrate 2, both probes D₁ and D₂ hybridize to the amplification product from individual 1, indicating a heterozygous individual, D₁D₂. Only probe D₁ hybridizes to the amplification product from individual 2, indicating a homozygous individual for allele D₁. Both probes D₁ and D₂ hybridize to the amplification product from individual 3, indicating a heterozygous individual, D₁D₂.

On substrate 3, only probe G₂ hybridizes to the amplification product from individuals 1 and 2, indicating homozygous individuals for allele G_{2.} Only probe G₁ hybridizes to the amplification product from individual 3, indicating a homozygous individual for allele G₁.

In the process described here, the presence or absence of each of the two polymorphic forms of marker is detected using a pair of fluorescently labeled probes. Each probe in a pair bears a different fluorescent label, indicated by star and dagger, that fluoresces a different, distinguishable color, e.g., blue (vertical hatching) and red (horizontal hatching), respectively. Referring again to Fig. 4, the first probe pair is directed to forms A₁ and A₂ of marker A. In this example, only blue light is detected after the hybridization of the "A" probes to feature 1 of substrate 1, indicating that only probe A₁ hybridized to the amplification product in this feature. Only red light is detected after hybridization of the "A" probes to feature 2 of substrate 1, indicating that only probe A₂ hybridized to the amplification product in feature 2 of substrate 1. Both red and blue light are detected after hybridization of the "A" probes to feature 3 of substrate 1, indicating that both probes A₁ and A₂ hybridized to the amplification product in feature 3 of substrate 1.

Thus, the signal generated by the fluorescent probes at any feature will indicate the genotype of the individual. Generally, interpreting a signal to indicate a particular genotype is carried out by a computer. The computer is programmed to correspond, for each marker, the color from a label with the presence of a particular allelic form of the marker.

### IV. ITERATIVE PROBING OF SINGLE SUBSTRATE FOR MULTIPLE MAKERS

Referring to Fig. 5, in order to determine the genotype of all of the amplified markers in an amplification product, the hybridization-detection process is iterated three times on a single substrate. Each time, the probes are directed to a different amplified marker in the amplification product. This figure shows only substrate 1 and individuals 1, 2 and 3. In the first iteration, already shown, probes are directed to marker A. After determining hybridization of the probes for each individual, the substrate is washed to remove any hybridized probes.

The substrate is probed again with probes to detect polymorphic forms of marker B. The results indicate that individual 1 has genotype B₂B₂, individual 2 has genotype B₁B₁, and individual 3 has genotype B₂B₂.

After detection of hybridization, the substrate is washed again and probed with probes to detect polymorphic forms of marker C. The results here indicate that individual 1 has genotype C₁C₂, individual 2 has genotype C₂C₂, and individual 3 has genotype C₂C₂.

### V. GENERATING A POLYMORPHIC PROFILE FOR MANY INDIVIDUALS

A multiplex polymorphic profile for an individual is created by assembling the genotypic data for a plurality of markers in an individual into a value set. A multiplex polymorphic profile of the population is created by assembling polymorphic profiles of all the individuals. Referring to Fig. 6, the assembled genotypes for markers A through I for each individual represents a multiplex polymorphic profile for the individual. The collection of multiplex polymorphic profiles from the many individuals results in the multiplex genotyping of the population.

The present invention provides novel materials and methods for multiplex polymorphic profiling of a population of individuals. While specific examples have been provided, the above description is illustrative and not restrictive. Many variations of the invention will become apparent to those skilled in the art upon review of this specification. The scope of the invention should, therefore, be determined not with reference to the above description, but instead should be determined with reference to the appended claims along with their full scope of equivalents.

All publications and patent documents cited in this application are incorporated by reference in their entirety for all purposes to the same extent as if each individual publication or patent document were so individually denoted. By their citation of various references in this document Applicants do not admit that any particular reference is "prior art" to their invention.

## Claims

1. A method of detecting a polymorphic form of a polymorphic marker in a plurality of individuals comprising:
a) producing a plurality of amplification products by performing a multiplex amplification on a nucleic acid sample from each of a plurality of individuals, each multiplex amplification amplifying a plurality of nucleic acid segments, each segment comprising a polymorphic marker characterized by at least two polymorphic forms;
b) applying each amplification product to a discrete region of a substrate; and
c) detecting the presence or absence of at least one polymorphic form of at least one polymorphic marker in each amplification product.

2. The method of claim 1 wherein detecting comprises detecting the presence or absence of a polymorphic form of a plurality of different polymorphic markers in each amplification product on the substrate in a plurality of sequential detection steps.

3. The method of claim 2 wherein step a) comprises dividing each sample into a plurality of fractions and performing a multiplex amplification on different polymorphic markers in each of the fractions.

4. The method of claim 2 wherein the plurality of nucleic acid segments is at least 100 segments.

5. The method of claim 2 wherein the plurality of individuals is at least 1000 individuals.

6. The method of claim 2 comprising applying the amplification products to the substrate in an orthogonal array.

7. The method of claim 2 wherein the amplification products are applied to the substrate by spotting or spraying.

8. The method of claim 2 wherein the substrate comprises oligonucleotide anchors, the segments comprise oligonucleotide tags that hybridize to the anchors, and the amplification products are applied to the substrate by hybridization.

9. The method of claim 1 wherein detecting comprises detecting a nucleic acid probe hybridized to a segment comprising the polymorphic marker.

10. The method of claim 2 wherein detecting comprises detecting a labeled oligonucleotide hybridized to the amplification product

11. The method of claim 2 further comprising the step of:
d) generating, for the plurality of individuals, a value set indicating the presence or absence of the polymorphic form, whereby the value set determines a polymorphic profile for the individuals.

12. The method of claim 2 wherein the at least one individual is human.

13. The method of claim 2 wherein the plurality of steps is at least 10.

14. The method of claim 5 comprising detecting the presence of at least 100 polymorphic markers.

15. The method of claim 9 wherein the probe is an allele-specific probe.

16. The method of claim 9 wherein the probe results from allele specific ligation.

17. The method of claim 9 wherein the probe results from chain extension termination.

18. The method of claim 10 wherein the label is a fluorescent label.

19. The method of claim 11 wherein the at least one polymorphic marker is a plurality polymorphic markers.

20. The method of claim 15 comprising hybridizing to each amplification product at least one pair of allele-specific nucleic acid probes, wherein each probe of the pair specifically hybridizes to an alternative, exclusively distinguishable polymorphic form of the selected polymorphic marker.

21. The method of claim 15 comprising hybridizing at least two allele-specific nucleic acid probes to the amplification product, wherein each of the probes specifically hybridizes to a segment comprising a different polymorphic marker.

22. The method of claim 20 wherein each of the pair of probes comprises a fluorescent label that emits light of a different wavelength.

23. The method of claim 20 comprising hybridizing a plurality of pairs and wherein each pair specifically hybridizes to a segment comprising a different polymorphic marker.

24. A kit comprising:
a) a plurality of primer pairs, each pair having sequences for amplifying a nucleic acid segment, wherein each segment comprises a different polymorphic marker characterized by at least two polymorphic forms; and
b) a set of allele-specific nucleic acid probes, wherein the set comprises, for each polymorphic marker, at least one probe that specifically hybridizes to a polymorphic form of the polymorphic marker.

25. The kit of claim 24 wherein the plurality of primer pairs is at least 1,000.

26. The kit of claim 24 wherein the plurality of primer pairs is at least 10,000.

27. The kit of claim 24 further comprising a substrate having a surface suitable for immobilizing the nucleic acid segments in an array.

28. The kit of claim 24 wherein the set of probes comprises, for each polymorphic marker, a pair of allele-specific probes, wherein each probe of the pair specifically hybridizes to an alternative, exclusively distinguishable polymorphic form of the polymorphic marker.

29. The kit of claim 28 wherein each probe of the pair comprises a fluorescent label that emits light of a different wavelength.

30. A kit comprising:
a) an array of amplification products, wherein each amplification product is the product of a multiplex amplification on a nucleic acid sample from each of a plurality of individuals, wherein each amplification product comprises a plurality of amplified nucleic acid segments, wherein each segment comprises a polymorphic marker characterized by at least two polymorphic forms; and
b) a set of allele-specific nucleic acid probes, wherein the set comprises, for each amplification product, at least one probe that specifically hybridizes to a polymorphic form of at least one polymorphic marker of an amplified segment.

31. The kit of claim 30 wherein each amplification product comprises at least 10 different amplified segments.

32. The kit of claim 30 wherein the set comprises, for at least one amplified segment of each amplification product, a pair of allele-specific probes, wherein each probe of the pair specifically hybridizes to an alternative, exclusively distinguishable polymorphic form of the polymorphic marker.

33. The kit of claim 32 wherein each probe of the pair comprises a fluorescent label that emits light of a different wavelength.

34. The kit of claim 31 wherein the array comprises amplification products for at least 1000 individuals.
